# EUROPEAN PATENT SPECIFICATION

(11) **EP 0 948 255 B1**
(45) Date of publication and mention of the grant of the patent: **19.10.2005**
(21) Application number: 97952253.9
(22) Date of filing: 05.12.1997
(51) Int. Cl.: A01N 1/02, A61K 35/28

(54) **IMPROVED CHONDROGENIC DIFFERENTIATION OF HUMAN MESENCHYMAL STEM CELLS**
VERBESSERTE CHONDROGENE DIFFERENZIERUNG VON MENSCHLICHEN MESENCHYM-STAMMZELLEN
PERFECTIONNEMENT CONCERNANT LA DIFFERENTIATION CHONDROGENE DE CELLULES SOUCHES DU MESENCHYME HUMAIN

(30) Priority: 06.12.1996 US 35274 P
(43) Date of publication of application: 13.10.1999
(73) Proprietor: Osiris Therapeutics, Inc., Baltimore, MD 21231-2001 (US)
(72) Inventor: PITTENGER, Mark, F., Severna Park, MD 21146 (US); MACKAY, Alastair, M., Timonium, MD 21093 (US); MURPHY, J., Mary, Baltimore, MD 21207 (US); BARRY, Francis, P., Baltimore, MD 21207 (US)
(74) Representative: LOUIS, PÖHLAU, LOHRENTZ
(86) International application number: PCT/US1997/022022
(87) International publication number: WO 1998/032333

(56) References cited:
- WO-A-96/23059
- WO-A-96/38168
- WO-A-96/39487
- WO-A-97/18299
- US-A- 5 226 914
- US-A- 5 466 572
- US-A- 5 486 359
- JOHNSTONE, B. ET AL: "In vitro chondrogenesis of bone marrow-derived mesenchymal cells" TRANS. ORTHOP. RES. SOC., February 1996 (1996-02), page 65-11 XP001098764
- CHIMAL-MONROY J. ET AL: "Regulation of chondrocyte differentiation by transforming growth factors Beta-1, beta-2, beta-3 and beta-5" ANNALS OF THE NERW YORK ACADEMY OF SCIENCE: DEVELOPMETAL BIOLOGY OF CARTILAGE, vol. 785, 1996, pages 241-244, XP001096298 new york
- KATO Y ET AL: "TERMINAL DIFFERENTIATION AND CALCIFICATION IN RABBIT CHONDROCYTE CULTURES GROWN IN CENTRIFUGE TUBES: REGULATION BY TRANSFORMING GROWTH FACTOR BETA AND SERUM FACTORS" PROCEEDINGS OF THE NATIONAL ACADEMY OF SCIENCES OF USA, NATIONAL ACADEMY OF SCIENCE. WASHINGTON, US, vol. 85, no. 24, December 1988 (1988-12), pages 9552-9556, XP001061768 ISSN: 0027-8424
- MINAMOTO Y ET AL: "DEVELOPMENT OF A SERUM-FREE AND HEAT-STERILIZABLE MEDIUM AND CONTINUOUS HIGH-DENSITY CELL CULTURE" CYTOTECHNOLOGY, KLUWER ACADEMIC PUBLISHERS, DORDRECHT, NL, vol. 2, 1991, pages S35-S51, XP000996331 ISSN: 0920-9069
- CELL TRANSPLANT., 09 May 1997, Vol. 6, No. 2, KADIYALA S. et al., "Culture Expanded Canine Mesenchymal Stem Cells Possess Osteochondrogenic Potential In Vivo and In Vitro", pages 125-134.
- J. CELL BIOL., June 1988, Vol. 106, GRIGORIADIS A.E. et al., "Differentiation of Muscle, Fat, Cartilage and Bone from Progenitor Cells Present in a Bone-Derived Clonal Cell Population: Effect of Dexamethasone", pages 2139-2152.
- ENDOCRINOLOGY, 1997, Vol. 138, No. 11, QUARTO R. et al., "Modulation of Commitment, Proliferation and Differentiation of Chondrogenic Cells in Defined Culture Medium", pages 4966-4976.
- SEM. IN CELL BIOL., 1995, Vol. 6, YAMAGUCHI A., "Regulation of Differentiation Pathway of Skeletal Mesenchymal Cells in Cell Lines by Transforming Growth Factor-beta Superfamily", pages 165-173.
- IN VITRO CELL. DEV. BIOL., September 1993, Vol. 29A, YOUNG H.E. et al., "Pluripotent Mesenchymal Stem Cells Reside Within Avian Connective Tissue Matrices", pages 723-736.

## Description

The present invention relates to the field of methods and compositions for directing mesenchymal progenitor cells cultivated *in vitro* to differentiate into specific cell lineage pathways, and particularly to such directed lineage induction prior to, or at the time of, their implantation into a recipient or host for the therapeutic treatment of pathologic conditions in humans and other species.

Mesenchymal stem cells (MSCs) are the formative pluripotent blast or embryonic-like cells found in bone marrow, blood, dermis, and periosteum that are capable of differentiating into specific types of mesenchymal or connective tissues including adipose, osseous, cartilaginous, elastic, muscular, and fibrous connective tissues. The specific differentiation pathway which these cells enter depends upon various influences from mechanical influences and/or endogenous bioactive factors, such as growth factors, cytokines, and/or local microenvironmental conditions established by host tissues. Although these cells are normally present at very low frequencies in bone marrow, a process for isolating, purifying, and mitotically expanding the population of these cells in tissue culture is reported in Caplan *et al*. U.S. Patent Nos. 5,197,985; 5,226,914; and 5,486,359. Mesenchymal stem cells. J. Orthoped. Res. 9: 641-650, 1991.

In prenatal organisms, the differentiation of MSCs into specialized connective tissue cells is well established; for example embryonic chick, mouse or human limb bud mesenchymal cells differentiate into cartilage, bone and other connective tissues (Caplan AI, Mesenchymal stem cells. J. Orthoped. Res. 9: 641-650, 1991. In: *39th Annual Symposium of the Society for Developmental Biology,* ed by S. Subtelney and U Abbott, pp 3768. New York, Alan R Liss Inc, 1981; Elmer *et al., Teratology, 24*:215-223, 1981; Hauschka SD, *Dev Biol, 37*:345-368, 1974; Solursh *et al., Dev Biol, 83*:9-19, 1981; Swalla *et al., Dev Biol, 116*:31-38, 1986). In addition, a clonal rat fetus calvarial cell line has also been shown to differentiate into muscle, fat, cartilage, and bone (Goshima *et al., Clin Orthop Rel Res, 269*:274-283, 1991). The existence of MSCs in post-natal organisms has not been widely studied with the objective of showing the differentiation of post-embryonic cells into several mesodermal phenotypes. The few studies which have been done involve the formation of bone and cartilage by bone marrow cells following their encasement in diffusion chambers and *in vivo* transplantation (Ashton *et al., Clin Orthop Rel Res, 151*:294-307, 1980; Bruder *et al., Bone Mineral, 11*:141-151, 1990). Recently, cells from chick periosteum have been isolated, expanded in culture, and, under high density conditions *in vitro,* shown to differentiate into cartilage and bone (Nakahara *et al., Exp Cell Res, 195*:492-503, 1991). Rat bone marrow-derived mesenchymal cells have been shown to have the capacity to differentiate into osteoblasts and chondrocytes when implanted *in vivo* (Dennis *et al., Cell Transpl, 1*:2332, 1991; *Goshima et al., Clin Orthop Rel Res, 269*:274-283, 1991). The phenotypes of chondrocytes in culture have been reported to be influenced by the concentrations of sugars available for glycolysis and for the citric acid cycle (P. Otte. "Basic cell metabolism of articular cartilage. Manometric studies." Z. *Rheumatol*. 50:304-12, 1991; and J. M. Lane, C.T. Brighton, and B.J. Menkowitz. "Anaerobic and aerobic metabolism in articular cartilage." *J Rheumatol.* 4:334-42, 1977).

Recently Johnstone *et al*. (Trans. Orthop. Res. Soc., 42:65, 1996) In vitro chondrogenesis of bone marrow-derived mesenchymal cells. Transactions of the Orthopaedic Research Society 21: 65; 1996. have described the culture conditions under which rabbit MSCs will develop a chondrocyte-like phenotype *in vitro.* The cells are grown in minimal culture conditions in the absence of serum but in the presence of dexamethasone, ITS, and ascorbic acid-phosphate. When the cells were spun at low speed they formed a layer that develops into a free-floating pellet within 1-2 days. Over a period of weeks the cells will begin to synthesize and secrete type II collagen. the original observations using human cells.

Damage to the articular surfaces of synovial joints can arise from trauma, from diseases such as osteoarthritis, and as a result of the aging process. The social and economic costs of damaged joints are large, and effective therapies that could restore joint function would be welcome. Articular cartilage is created and maintained during prenatal and postnatal growth by mesenchymal cells that have differentiated into articular chondrocytes. Individuals may lose the ability to repair major synovial defects as they mature because their joints lack sufficient numbers of properly-differentiated cells to regenerate articular cartilage. Thus, there has been a great deal of interest in the hypothesis that damaged joint surfaces may be repaired by implanting autologous cells that will reconstitute a suitable extracellular matrix. One study involving the introduction of cultured chondrocytes into the knee appeared to have great promise ¹. As orthopedic surgeon Joseph Buckwalter pointed out in a recent essay, this and similar efforts have met with equivocal long-term success (J.A. Buckwalter. "Regenerating articular cartilage: Why the sudden interest?" *Orthopedics Today.* April 12, 1996).

Thus, there is a continuing need and opportunity for cartilage-regeneration therapies.

WO 96/23059 discloses Dulbecco's Modified Eagle's Medium-Low Glucose for culturing human mesenchymal stem cells.

### Summary of the Invention

To date, it has not been possible to cause large numbers of hMSCs to uniformly commit to the chondrocytic lineage. The composition used in the process pursuant to claim 1 or 12 accomplishes this goal. Thus, this invention represents an important step in the development of a technology , autologous MSC-based repair of articular cartilage, that has widespread and significant potential utility.

In accordance with the present invention it has been observed by the inventors that human mesenchymal stem cells (hMSCs) maintain their viability and can be induced to significantly improved commitment and differentiation when contacted *in vitro* with certain chondroinductive media compositions having elevated levels of simple sugars or other factors which contribute to the production of ATP by the citric acid cycle as specified in claim 1 or 12. The hMSCs are associated in a three-dimensional format, such as a cell pellet, in the process as claimed in claim 1 or 12. The three dimensional format contributes to the *in vitro* chondrogenesis of the invention and the cells are preferably condensed together, for example, as a packed or pelleted cell mass. This *in vitro* process is believed to recapitulate that which occurs *in vivo* and can be used to define the molecular events that are important in the process of chondrogenesis.

Thus, in one aspect the invention provides a process pursuant to claim 1 or 12 using a composition for the *in vitro* chondrogenesis of human mesenchymal precursor cells and the *in vitro* formation of human chondrocytes therefrom, which composition comprises isolated human mesenchymal stem cells (in a three dimensional format) and at least one chondroinductive agent in a medium having a simple sugar concentration of from at least about 3 grams/liter (g/l), preferably from about 3 g/l to about 7 g/l in contact therewith. The mesenchymal stem cells are preferably isolated, culture expanded human mesenchymal stem cells in a chemically defined serum-free environment and can be condensed into close proximity, such as in the form of a three dimensional cell mass, *e.g.* packed cells or a centrifugal cell pellet.

In another aspect of the invention it has been discovered that TGF-β3 is a more effective chondroinductive agent than those previously used such as *(i)* a glucocorticoid such as dexamethasone; *(ii)* other members of the transforming growth factor-β superfamily such as a bone morphogenic protein (preferably BMP-2 or BMP-4), TGF-β1, inhibin A or chondrogenic stimulating activity factor; *(iii)* a component of the collagenous extracellular matrix such as collagen **I**; or *(iv)* a vitamin A analog such as retinoic acid. The TGF-β3 is included in the medium in an amount effective to induce differentiation of MSCs predominantly into chondrocytes. Such a concentration is at least about 5 ng/ml of medium, preferably 5-15 ng/ml of medium.

The invention also provides a process for producing chondrocytes from mesenchymal stem cells by contacting mesenchymal stem cells with a chondroinductive agent *in vitro* in the above-described improved medium as specified in claim 1, particularly one with a glucose or lactose concentration higher than that previously used in inducing chondrogenic differentiation.

The invention also provides a process for inducing chondrogenesis in mesenchymal stem cells by contacting mesenchymal stem cells with the composition of the invention *in vitro* as specified in claim 12.

Further disclosed herein are *in vitro* culture conditions that allow and promote the further differentiation and maturation of hMSC-derived chondrocytes to hypertrophic chondrocytes. This is useful, *inter alia*, for (i) studies of factors involved in chondrocyte maturation, (ii) studies of alterations in gene expression that occur during chondrogenesis, (iii) identification and study of factors produced by maturing chondrocytes, (iv) evaluation of the effects of pharmacological agents on maturing chondrocytes, (v) determination of the susceptibility of maturing chondrocytes to matrix-metaloproteinases and other degradative enzymes common in joint diseases such as osteoarthritis and (vi) studies of the expression of genes introduced into hMSCs for the purpose of ameliorating joint disease.

The ability of hMSCs to undergo hypertrophic differentiation has direct relevance for the repair of full-thickness defects of articular cartilage by implanted hMSCs. The implant is better 'anchored' to adjoining tissue, and the repair made more permanent, when hypertrophic cartilage, mineralized cartilage, and bone replaced the implanted material in the deep areas of the defect. The *in vitro* results presented here are an important indication that such replacement is a viable clinical option. Thus, part of this aspect of the invention has been to modulate the chondrogenic differentiation process by identifying conditions that promote a specific phenotype, *i.e.* to direct the cells to become hypertrophic chondrocytes, since that phenotype is pronounced, and indicates the maturation of the cells beyond the chondroblast stage.

In the above methods, the mesenchymal stem cells are preferably isolated, culture expanded human mesenchymal stem cells in a chemically defined serum-free environment and are condensed into close proximity, such as in the form of a three dimensional cell mass, *e.g.* packed cells or a centrifugal cell pellet. Further, the contacting preferably comprises culturing a pellet of human mesenchymal precursor cells in a chemically defined serum-free medium as defined in claim 1 or 12. The above methods can also preferably comprise steps where the cells are cultured with the chondroinductive composition and thereafter placed in a rigid porous vessel, such as a ceramic cube.

It is also possible to use an isolated, non-cultured non-homogeneous human mesenchymal stem cell preparation in the composition and methods of the invention. MSCs can be isolated as non-cultured, non-homogeneous preparations, such as by density gradient fractionation, from tissue such as bone marrow, blood (including peripheral blood), periosteum and dermis, and other tissues which have mesodermal origins. In this regard, it has been found that although these mesenchymal stem cells are normally present in bone marrow, for example, in very minute amounts and that these amounts greatly decrease with age (*i.e.* from about 1/10,000 cells in a relatively young patient to as few as 1/2,000,000 in an elderly patient), human mesenchymal stem cell preparations can be isolated from tissue, particularly bone marrow, so as to be substantially free of other types of cells in the marrow. It is contemplated that the isolated fractionation preparation will comprise cells of which at least about 90%, and preferably at least about 95 %, are human mesenchymal stem cells.

Further disclosed are *in vitro* culture conditions that allow and promote the further differentiation and maturation of hMSC-derived chondrocytes to hypertrophic chondrocytes. This proves useful in studies of factors involved in chondrocyte maturation, studies of alterations in gene expression that occur during chondrogenesis, identification and study of factors produced by maturing chondrocytes, evaluation of the effects of pharmacological agents on maturing chondrocytes, determination of the susceptibility of maturing chondrocytes to matrix-metaloproteinases and other degradative enzymes common in joint diseases such as osteoarthritis, studies of the expression of genes introduced into hMSCs for the purpose of ameliorating joint disease.

The sequence of events that occur in the induction of chondrogenesis and production of chondrocytes in the above *in vitro* methods resembles that of chondrogenesis in embryonic limb formation. Since all components of the system are defined, the system can be used as a valuable research tool for studies of the effects of growth factors *etc*. on the progression of chondrogenesis. It is also applicable to studies of the molecular control of mammalian chondrogenesis from progenitor cells.

### Brief Description of the Drawings

The invention will now be further described by reference to a brief description of each of the Figures, which are in no way are a limitation of the scope of the invention.

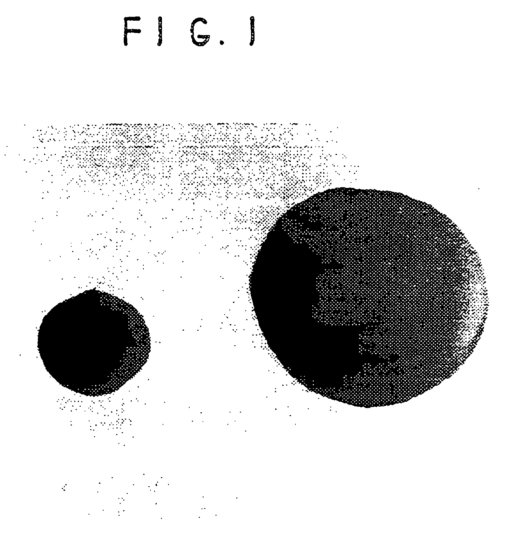

**Figure 1**. Changes in the size of hMSC pellets reflect the extent of synthesis of extracellular matrix. components. Each pellet was formed by transferring an aliquot of 200,000 cells into ½ ml chondrogenic medium with 1 g/l (5.5 mM) glucose (left) or 4.5 g/l (25 mM) glucose (right).

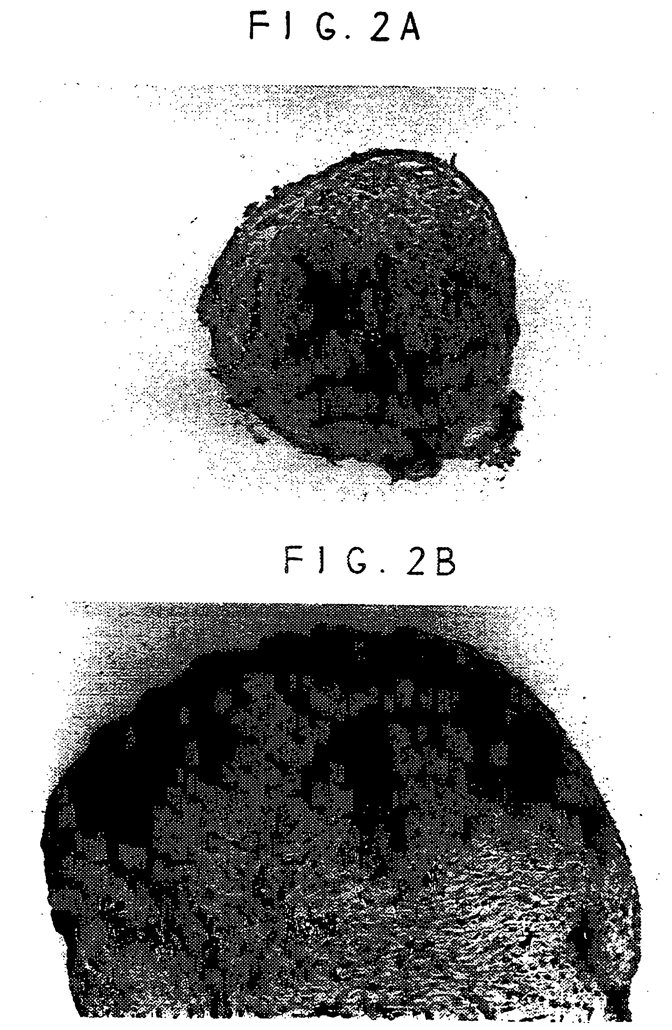

**Figure 2A**. 8 µm sections of hMSC pellets grown in chondrogenic medium with 1 g/l glucose after one week of culture. Sections were immunostained for the presence of type II collagen and developed, but did not reveal the brown reaction product, seen in the pellet grown with high glucose as shown in Figure 2B. Sections were also stained with hematoxylin. Magnification, 125x.
**Figure 2B.** 8 µm sections of hMSC pellets grown in chondrogenic medium with 4.5 g/l glucose after one week of culture. Sections were immunostained for the presence of type II collagen, and developed to reveal a brown reaction product, seen only in the pellet grown with high glucose. Sections were also stained with hematoxylin. Magnification, 125x.

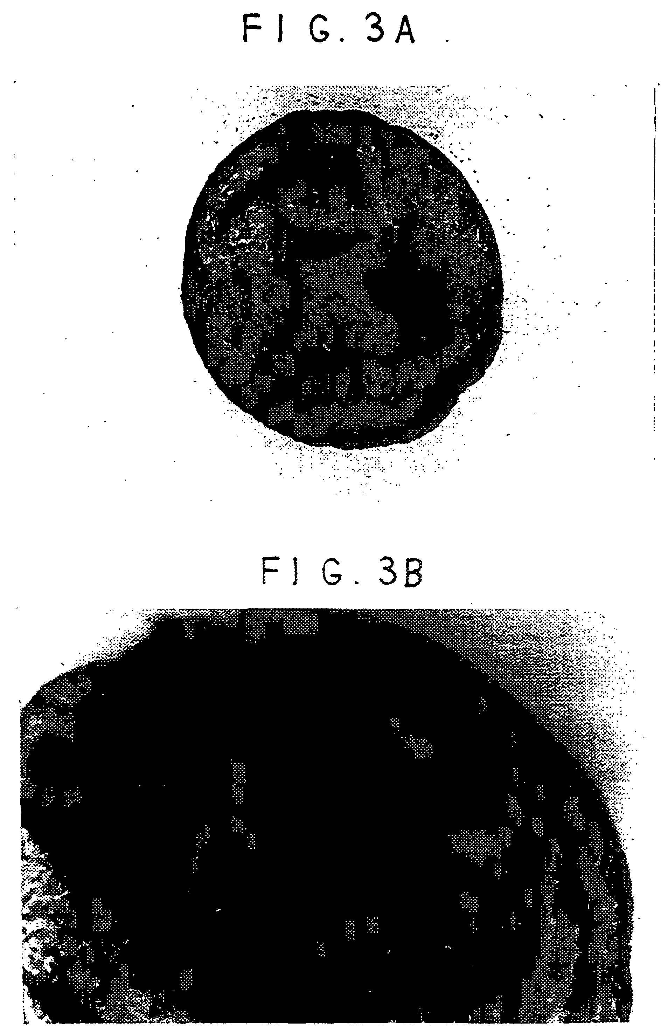

**Figure 3A.** Thin sections of hMSC pellets grown in low-glucose medium for two weeks. Immunostaining shows that the pellet in the low-glucose conditions had accumulated less type II collagen than the pellet in the high-glucose conditions shown in Figure 3B. Magnification, 125x.
**Figure 3B** Thin sections of hMSC pellets grown in high-glucose medium for two weeks. Immunostaining shows that the pellet in the high-glucose conditions had accumulated more type II collagen than the pellet in the low-glucose conditions shown in Figure 3A. Magnification, 125x.

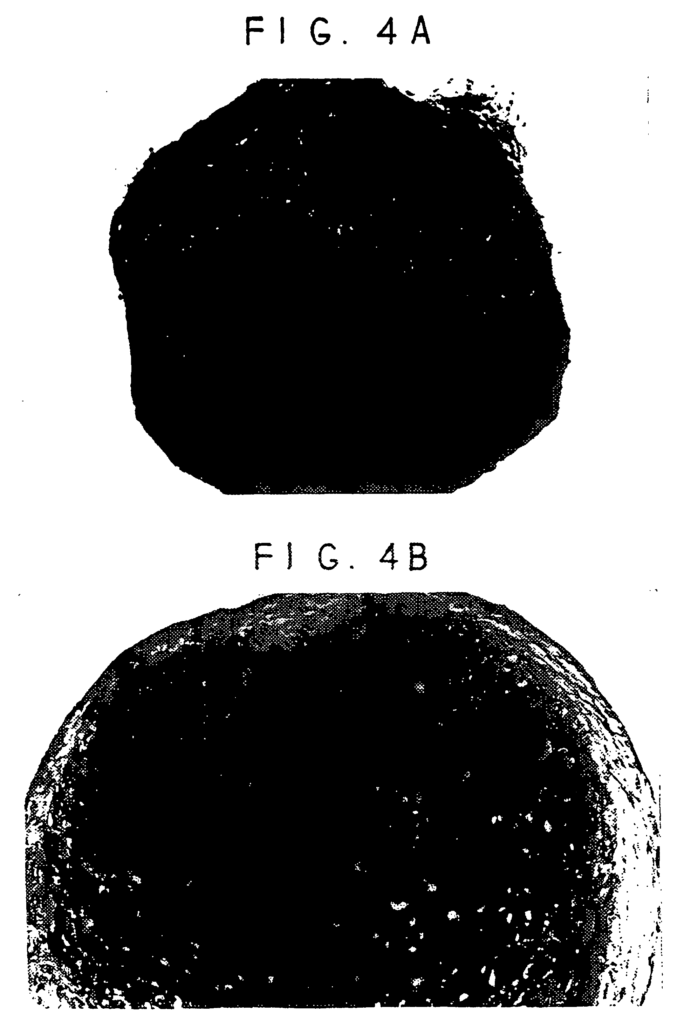

**Figure 4A.** Thin sections of hMSC pellets stained with toluidine blue O after three weeks of growth. The purple 'metachromatic' staining characteristic of a cartilaginous extracellular matrix is less prominent than in the high-glucose pellet shown in Figure 4B. Magnification, 125x.
**Figure 4B.** Thin sections of hMSC pellets stained with toluidine blue O after three weeks of growth. The purple 'metachromatic' staining characteristic of a cartilaginous extracellular matrix is more prominent in the high-glucose pellet (**B**) than in the low-glucose pellet shown in Figure 4A. Magnification, 125x.

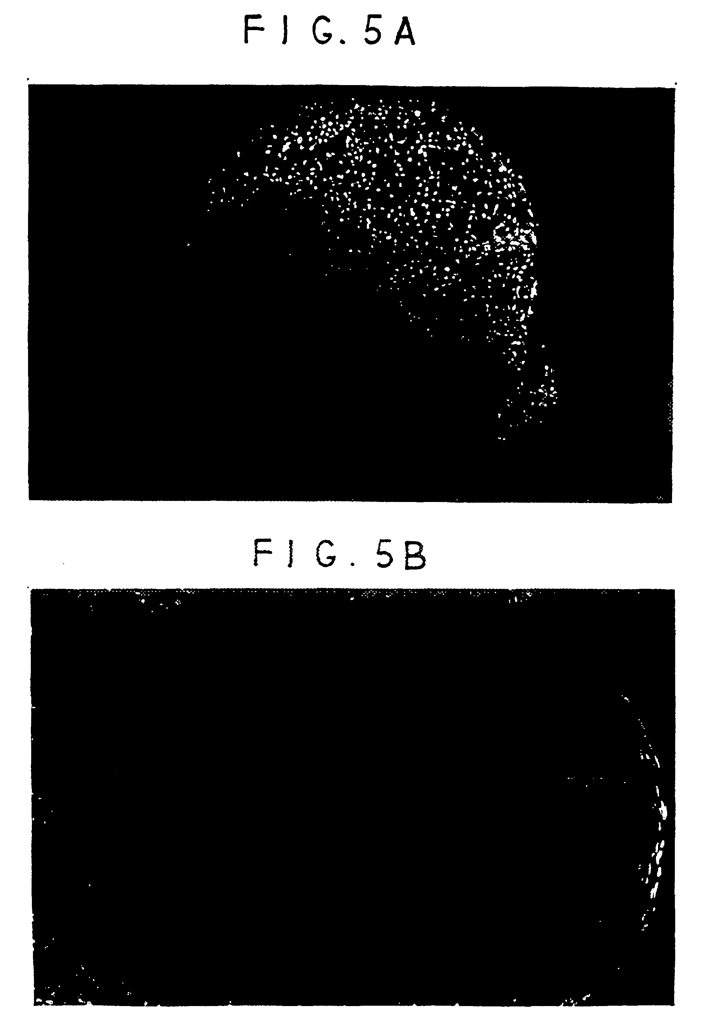

**Figure 5A.** Viability staining of 21-day-old hMSC pellets. Cultured pellets were incubated with 2 µM ethidium homodimer dye for 72 hr, then fixed, sectioned, and post-stained with a second dye, DAPI. The nuclei of non-viable cells incorporate ethidium homodimer and thus fluoresce red. Viable cells fluoresce blue with DAPI incorporated after fixation and sectioning. Cell death is a prominent feature of low-glucose pellets, as can be seen here. Magnification, 125x
**Figure 5B.** Viability staining of 21-day-old hMSC pellets. Cultured pellets were incubated with 2 µM ethidium homodimer dye for 72 hr, then fixed, sectioned, and post-stained with a second dye, DAPI. The nuclei of non-viable cells incorporate ethidium homodimer and thus fluoresce red. Viable cells fluoresce blue with DAPI incorporated after fixation and sectioning. Cell death is a prominent feature of low-glucose pellets, and much reduced by culture in high-glucose conditions, as compared to Figure 5A. Magnification, 125x.

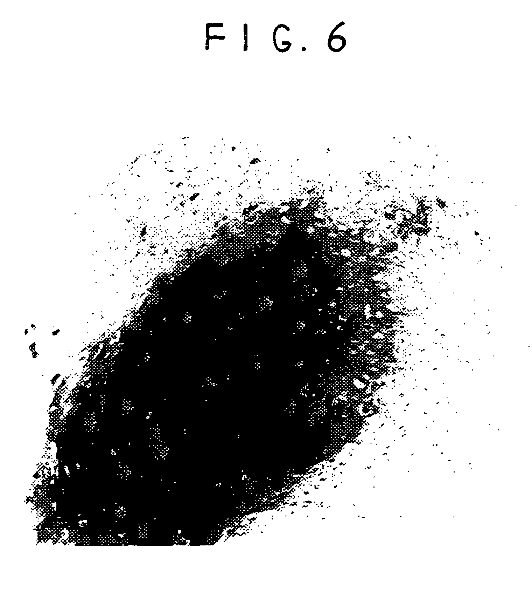

**Figure 6** shows pellets of hMSCs, prepared as described in Example 4, in chondrogenic culture which contained regions of cells with a hypertrophic morphology. Thus, hMSCs can undergo hypertrophy under culture conditions for chondrogenic differentiation. As shown, an hMSC pellet underwent incomplete chondrogenic differentiation after 21 days in culture. High-glucose DMEM was supplemented with TGF-β3, dexamethasone, and other agents, as described. The section was immunostained for type II collagen. The incomplete brown stain indicated that only part of the pellet contained cells that secreted this marker of cartilage. However, this type-II-collagen-positive region contained cells within large lacunae. Such morphology suggested that hMSCs in pellet culture are capable of undergoing hypertrophic differentiation. Final magnification, 150x.

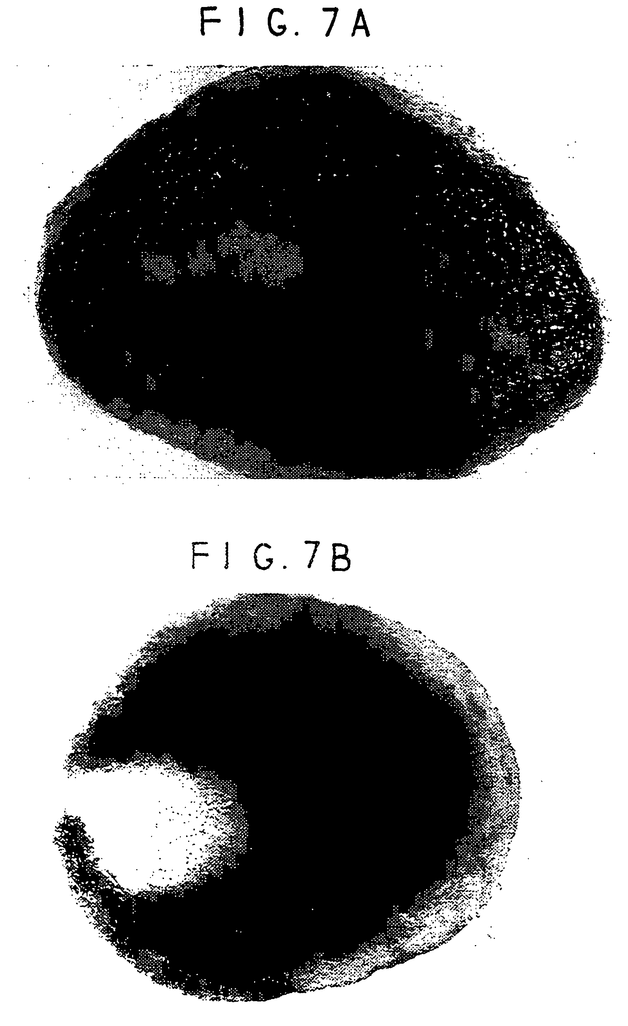

**Figures 7A and 7B** also show pellets of cells, prepared as described in Example 5, which showed much greater hypertrophicity than those of Fig. 6. These Figures show that sequential treatment of hMSCs in pellet culture with two media induces hypertrophy. After 14 days of culture in chondrogenic medium, the pellet of Figure 7A was switched to medium lacking TGF-β3, and containing 10⁻⁹ M dexamethasone, 50 nM thyroxine, and 20 mM β-glycerol phosphate. The pellet of Figure 7B remained in the original medium. At 28 days, both pellets were harvested, sectioned, and stained with antibody against type II collagen. Type II collagen is prominent in both pellets. The extensive number of cells within enlarged lacunae in the pellet of Figure 7A indicated that hypertrophic differentiation of hMSCs was the predominant pathway in this two-stage culture regimen. In contrast, the pellet of Figure 7B had relatively few hypertrophic chondrocytes. Final magnification, 60x.

### Detailed Description of Preferred Embodiments

The invention will now be described in more detail with respect to numerous embodiments and examples in support thereof.

Mesenchymal stem cells (MSCs) are the formative pluripotent blast or embryonic-like cells found in bone marrow, blood, dermis, and periosteum that are capable of differentiating into specific types of mesenchymal or connective tissues including adipose, osseous, cartilaginous, elastic, muscular, and fibrous connective tissues. The specific differentiation pathway which these cells enter depends upon various influences from mechanical influences and/or endogenous bioactive factors, such as growth factors, cytokines, and/or local microenvironmental conditions established by host tissues. Although these cells are normally present at very low frequencies in bone marrow, a process for isolating, purifying, and mitotically expanding the population of these cells in tissue culture is reported in Caplan *et al*. U.S. Patent Nos. 5,197,985; 5,226,914; and 5,486,359. Mesenchymal stem cells. J. Orthoped. Res. 9: 641-650, 1991.

Human mesenchymal stem cells are capable of producing multiple types of mesenchymal cells, and in particular cartilage. This trait, along with two others, makes these cells attractive candidates for use in autologous cell therapy for the repair of articular surfaces. First, there is the relative ease with which human MSCs may be obtained from bone marrow aspirates. Second, these cells have a demonstrated ability to undergo expansion many thousand-fold in culture (S.P. Bruder, N. Jaiswal, and S.E. Haynesworth. "Growth kinetics, self-renewal and the osteogenic potential of purified human mesenchymal stem cells during extensive subcultivation and following cryopreservation." *Journal of Cellular Biochemistry,* 1996, in press).

Described herein is an improvement over current methods used to induce chondrogenic differentiation of primary and passaged human mesenchymal stem cells (hMSCs) *in vitro.* This improvement builds on the "pellet culture" tissue culture protocol that was developed to promote the re-differentiation of cultured chondrocytes (Y. Kato, M. Iwamoto, T. Koike, F. Suzuki, and Y. Takano. "Terminal differentiation and calcification in rabbit chondrocyte cultures grown in centrifuge tubes: regulation by transforming growth factor beta and serum factors. " *Proc. Natl. Acad. Sci. USA* 85:9552-56, 1988; R.T. Ballock and A.H. Reddi. "Thyroxine is the serum factor that regulates morphogenesis of columnar cartilage from isolated chondrocytes in chemically defined medium." *J. Cell Biol.* 126:1311-18, 1994; and C. Xu, B.O. Oyajobi, A. Frazer, L.D. Kozaci, R.G.G. Russell, and A.P. Hollander, Effects of growth factors and interleukin-1α on proteoglycan and type II collagen turnover in bovine nasal and articular chondrocyte pellet cultures." *Endocrinology* 137:3557-65, 1996). We also describe an assay to assess cell viability that proves the utility of our culture method. These improvements allow for further discoveries relating to the differentiation of hMSCs, including the identification of genes related to novel therapeutic modalities.

In the experiments reported here, we show that increasing the glucose concentration of chondrogenic medium from the standard concentration present in "low glucose DMEM" (1 g/l, 5 mM) to the amount present in "high glucose DMEM" (4.5 g/l, 25 mM) dramatically alters the differentiation of cultured hMSCs. The effect of high concentrations of other sugars on chondrogenesis was also investigated. Supplementation of low-glucose medium with either 3.5 g/l fructose or with 6 g/l glucose yields the same improvement to *in vitro* chondrogenic differentiation as does high-glucose medium (4.5 g/l glucose).

This invention has multiple uses and advantages. One such advantage lies in the ability to direct and accelerate MSC differentiation prior to implantation back into autologous hosts. For example, MSCs which are directed *in vitro* to become chondrogenic cells will synthesize cartilage matrix at an implant site more rapidly and uniformly than MSCs which must first be recruited into the lineage and then progress through the key differentiation steps. Such an *ex vivo* treatment also provides for uniform and controlled application of bioactive factors to purified MSCs, leading to uniform lineage commitment and differentiation. *In vivo* availability of endogenous bioactive factors cannot be as readily assured or controlled. A pretreatment step such as is disclosed herein circumvents this. In addition, by pretreating the MSCs prior to implantation, potentially harmful side effects associated with systemic or local administration of exogenous bioactive factors are avoided. Another use of this technique lies in the ability to direct tissue regeneration based on the stage of differentiation which the cells are in at the time of implantation. That is, with respect to cartilage, the state of the cells at implantation may control the ultimate tissue type formed.

As used herein the term "simple sugar" refers to aldoses such as D-glucose, D-mannose and D-galactose and ketoses such as D-fructose.

As used herein the terms "chondroinductive agent" or "chondroinductive factor" refers to any natural or synthetic, organic or inorganic chemical or biochemical compound or combination or mixture of compounds, or any mechanical or other physical device, container, influence or force that can be applied to human mesenchymal stem cells which are in a three dimensional format so as to effect their *in vitro* chondrogenic induction or the production of chondrocytes. Known chondroitiductive agents are, for eicarnple, *(i)* a glucocorticoid such as dexamethasone; *(ii)* a member of the transforming growth factor-β superfamily such as a bone morphogenic protein (preferably BMP-2 or BMP-4), TGF-β1, inhibin A or chondrogenic stimulating activity factor (CSA); *(iii)* a component of the collagenous extracellular matrix such as collagen I (particularly in the form of a gel); and *(iv)* a vitamin A analog such as retinoic acid.

As used herein the term "chemically defined medium" refers to a maintenance, growth or culture medium in which the composition of the invention can undergo in vitro chondrogenesis, particularly in accordance with the methods of the invention, and includes a minimum essential medium, ascorbate, an iron source, insulin or an insulin-like growth factor, at least one chondroinductive agent or factor, and a simple sugar, said simple sugar being present in said medium in an amount of from 3 g/l to 7 g/l.

As used herein the term "minimum essential medium" refers to any serum-free animal cell culture preparation or medium of known composition which will support the viability of human mesenchymal stem cells *in vitro*. Examples are any of the Eagle's based media, *i*.*e*., Dulbecco's Modified Eagle's Medium (DMEM); Iscove's Modified Eagle's Medium, alpha Modified Eagle's Medium, and also McCoy's 5A and BGJ_{b} (Fitton-Jackson Modification).

As used herein the term "iron source" refers to any species that will release the reduced, ferrous form of iron to the medium, including but not limited to transferrin, FeSO₄ or ferritin.

As used herein the term "insulin" refers to any of the various insulins that are known. Insulins are divided into three categories according to promptness, duration and intensity of action following subcutaneous administration, *i*.*e*., as mentioned above, rapid, intermediate or long-acting. Crystalline regular insulin is prepared by precipitation in the presence of zinc chloride and modified forms have been developed to alter the pattern of activity. Protamine zinc insulin (PZI) is the result of the reaction of insulin and zinc with the basic protein, protamine, to form a protein complex which dissolves and is absorbed more slowly than crystalline regular insulin but is highly reliable for absorption at a steady rare. Isophane is a modified crystalline protamine zinc insulin whose effects are comparable to a mixture of predominantly regular insulin with a lesser portion of protamine zinc insulin. The extended and prompt insulin-zinc suspensions are also contemplated for use in the invention. The insulin can be, for example, of human bovine, ovine or other animal origin or can be a recombinant product.

Human insulin is now widely available as a result of its production by recombinant DNA techniques; in theory it should be slightly less immunogenic than purified porcine insulin, which in turn should be less immunogenic than bovine insulin. Bovine insulin differs from human insulin by three amino acid residues, whereas porcine differs from human insulin by only one amino acid at the carboxyl-terminus of the β-chain. However, when highly purified, all three insulins have a relatively low, but measurable, capacity to stimulate the immune response.

Short- or rapid-acting insulins are simply solutions of regular, crystalline zinc insulin (insulin injection) dissolved in a buffer at neutral pH. These have the most rapid onset of action but the shortest duration, *i*.*e*., glucose levels reach a low point within 20-30 minutes and return to baseline in about 2-3 hours.

Intermediate-acting insulins are formulated so that they dissolve more gradually when administered subcutaneously; their durations of action are thus longer. The two preparations most frequently used are neutral protamine Hagedorn (NPH) insulin (isophane insulin suspension) and Lente insulin (insulin zinc suspension). NPH insulin is a suspension of insulin in a complex with zinc and protamine in a phosphate buffer. Lente insulin is a mixture of crystallized (Ultralente) and amorphous (Semilente) insulins in an acetate buffer, which minimizes the solubility of insulin. The preparations have similar pharmacokinetic profiles.

Ultralente insulin (extended insulin zinc suspension) and protamine zinc insulin suspension are long-acting insulins; they have a very slow onset and a prolonged ("flat") peak of action. These insulins are advocated to provide a low basal concentration of insulin throughout the day.

As used herein the term insulin is also contemplated to encompass insulin analogs. Recent development of insulin that have altered rates of absorption have raised interest. Insulin with aspartate and glutamate substituted at positions B9 and B27, respectively, crystallizes poorly and has been termed "monomeric insulin". This insulin is absorbed more rapidly from subcutaneous depots and thus may be useful in meeting postprandial demands. By contrast, other insulin analogs tend to crystallize at the site of injection and are absorbed more slowly. Insulins with enhanced potency have been produced by substitution of aspartate for histidine at position B10 and by modification of the carboxyl-terminal residues of the B chain.

An example of the components of the chondrogenic media of the invention are shown in Table 1.

**Table 1**

| **Composition of Chondrogenic Medium Used In These Experiments** | | | | |
|---|---|---|---|---|
| *Ingredient* | *Supplier* | *Stock* | *Dilution* | *Final concentration* |
| DMEM (high glucose) | GIBCO/BRL | as supplied | none | neat |
| ITS+ supplement | Collaborative | as supplied | 1:99 | 6.25 µg/ml bovine insulin |
| Dexamethasone | Sigma | I mM in EtOH | 2 x 1:99 | 100 nM |
| Transforming Growth Factor | Calbiochem | 40 µg/ml | 1:4000 | 10 ng/ml |
| Ascorbic acid-2-phosphate | Wako | 5 mg/ml | 1:99 | 5 µg/ml |
| Sodium pyruvate | GIBCO/BRL | 100 mM | 1:99 | 1 mM |
| Proline | Sigma | 4 mg/ml | 1:99 | 40 µg/ml |
| Antibiotic-antimycotic | GIBCO/BRL | as supplied | 1:99 | 100 U/ml penicillin |

### Example 1

### High Glucose Medium Increases Extracellular Matrix

### Production During Chondrogenic Differentiation

When hMSCs differentiate down the chondrogenic lineage, cell metabolism is altered, and the anabolic activities of the cell are altered. One manifestation of this is the increase in extracellular matrix production. It is this extracellular matrix that is responsible for the unique properties of chondrocytes and allows them to serve as a weight bearing, lubricating tissue between adjoining bones, and it is this surface that is diseased in osteoarthritis. The extracellular matrix is composed of proteins and sulfated proteoglycans which are expressed in a temporal fashion as the cell develops into a chondrocyte. The proteins and proteoglycans include aggrecan, cartilage oligomatrix protein (COMP), hyaluronic acid, keratan sulfate, link protein and collagen type II as well as others. These molecules are assembled extracellularly and surround the cell body.

The hMSCs in pellet culture that are undergoing chondrogenic differentiation express the extracellular matrix proteoglycans mentioned above. In this disclosure, we show that the medium containing 4.5 g/l of glucose results in greater cell viability and greater production of extracellular matrix components as shown. In **Figure 1,** the increase in the size of the cell pellet is evident when the medium contains "high glucose", Changes in the size of hMSC pellets reflect the extent of synthesis of extracellular matrix. components. Each pellet was formed by transferring an aliquot of 200,000 cells into ½ ml chondrogenic medium with 1 g/l (5.5 mM) glucose (left) or 4.5 g/l (25 mM) glucose (right). The chondrogenic medium consisted of DMEM with the stated concentration of glucose and the following supplements: 100 nM dexamethasone, 10 ng/ml Transforming Growth Factor β, 1 mM sodium pyruvate, 5 µg/ml ascorbic acid-2-phosphate, and 40 µg/ml proline. A 1:99 dilution of "ITS+" supplied 6.25 µg/ml bovine insulin, 6.25 µg/ml transferrin, 6.25 µg/ml selenous acid, 5.33 µg/ml linoleic acid, and 1.25 mg/ml bovine serum albumin. Final concentrations of 100 U/ml penicillin, 100 µg/ml streptomycin, and 250 ng/ml amphotericin B were supplied by a 1:99 dilution of a concentrated antibiotic solution.

The cells and medium were placed into a 15 ml polypropylene conical-bottom centrifuge tube, and gently centrifuged (500 x g for 5 min.), after which the tubes with loosened caps were placed in a humidified 5 % CO2 incubator at 37 C. Over the ensuing 12 hours, the cells at the bottom of the tube reorganized themselves into a spherical pellet with a diameter of about 1 mm.

The "pellet cultures" were maintained by supplying the cells with fresh medium. Three times a week, medium was aspirated from the tube and 0.5 ml of fresh medium was added. The tube was gently shaken to make sure the pellet was free floating and not attached to the side of the tube. For Figure 1, pellets were fixed, after two weeks' growth, for 60 min in 4% paraformaldehyde in phosphate-buffered saline and photographed prior to embedding and freezing. The pellet formed by hMSCs in chondrogenic medium with 1 g/l glucose is 0.8 mm in diameter (left). The pellet cultured with 4.5 g/l glucose has a diameter of 1.6 mm (right). The increased size of the hMSC pellet in medium containing 4.5 g/l glucose is due to the large amount of extracellular matrix produced, rather than due to increased cell proliferation. Final magnification, 40x. To examine different chondrogenic conditions, the cell pellets were fixed, sectioned at 5-8 microns, and subjected to histological and immunohistological staining.

As shown in **Figure 2**, the onset of expression of type II collagen characteristic of cartilage was more advanced by as much as one week in cells cultured in chondrogenic medium containing 4.5 g/l high-glucose medium. For example, hMSCs showed little evidence of expression of this protein after the first week of culture in chondrogenic medium with 1 g/l glucose (**Figure 2A**). In contrast, the onset of type II collagen synthesis was readily detected within pellets grown in the chondrogenic medium with 4.5 g/l glucose **(Figure 2B**). At the two-week time point, type II collagen was present in restricted areas in pellets grown in media with 1 g/l glucose (**Figure 3A**), while evidence of type II collagen synthesis was clearly detected throughout the pellets grown with 4.5 g/l glucose (**Figure 3B**).

Formation of extracellular matrix with a high concentration of sulfated proteoglycans was demonstrated by staining with Safranin O (not shown) and toluidine blue O. When stained with toluidine blue O, sections of pellets with a cartilaginous extracellular matrix exhibit metachromasia, a purplish rather than blue color that demonstrates the presence of negatively-charged matrix elements, such as those found in cartilage and mentioned above. While hMSC pellets in chondrogenic medium with 1 g/l glucose showed evidence of this purple metachromatic stain after two weeks of culture (**Figure 4A**) when sectioned and stained, the extent of this staining was much more prominent when the culture medium contained 4.5 g/l glucose (**Figures 4B**).

### Example 2

### High Glucose Increases hMSC Viability During Chondrogenesis

There are a number of possible explanations for the improved chondrogenic differentiation of hMSCs cultured in medium containing 4.5 g/l glucose. However, this Example shows that this higher-glucose medium promotes cell survival to a much greater extent than does medium with 1 g/l glucose. High viability may result in greater chondrogenesis simply because a larger number of living cells are able to secrete cartilaginous extracellular matrix components. In addition, the greater levels of cell survival appear to be reflective of a more robust population of cells, where each living cell contributes more extracellular matrix protein, proteoglycan, and carbohydrate to its neighborhood. Because cartilage is an avascular organ, this requirement for a relatively high concentration of sugar in the culture medium may be reflective of the unusual metabolic characteristics of chondrocytes *in vivo.*

The viability of hMSCs in pellet culture was assayed by modifying the dye-exclusion assay developed by Poole et al. (C.A. Poole, N.H. Brookes, R.T. Gilbert, B.W. Beaumont, A. Crowther, L. Scott, and M.J. Merrilees. "Detection of viable and non-viable cells in connective tissue explants using the fixable fluoroprobes 5-chloromethylfluorescein diacetate and ethidium homodimer-1." *Connective Tissue Research* 33:233-241, 1996) for examining the survival of cells in organ culture. Seventy-two hours prior to the harvest of pellets, ethidium homodimer dye (1 mM stock in DMSO) was added to media to a final concentration of 2 µM. Pellets were then returned to standard incubation conditions. At harvest, pellets were rinsed 4 x 30 min. in phosphate-buffered saline, then fixed for 1 hr. in 4% paraformaldehyde, embedded in frozen-section embedding solution, cooled in a liquid nitrogen bath, and cryo-sectioned. Eight-µm sections were counter-stained with 500 ng/ml 4,6-diamidino-2-phenylindole (DAPI) prior to aqueous mounting and observation by fluorescence microscopy.

Under these conditions of ethidium homodimer dye incubation, only those cells without intact plasma membranes allowed the entry of the dye into their nuclei. These nuclei fluoresce red when excited with 490 nm light. Incubation of the fixed sections with the blue-fluorescing DAPI (1 µg/ml) allows the intercalation of this second dye into the DNA of all other nuclei, where the intra-helix binding sites are not occupied by ethidium homodimer. Thus, the nuclei of cells that were viable at the time of ethidium homodimer incubation and fixation glow red, whereas viable cells glow blue under observation by epi-fluorescence microscopy.

Examination of sections of ethidium-homodimer-treated pellets reveal that the reddish nuclei of non-viable cells are predominant in pellets grown with 1 g/l glucose (**Figure 5A**). In marked contrast, equivalent pellets grown with 4.5 g/l glucose contain few reddish nuclei (**Figure 5B**).

### Example 3

### TGF-β3 Is A Superior Chondrogenic Agent for MSCs

The experiments reported here investigated the chondrogenesis of MSCs from a relatively large number of rabbits (approximately 16), under a variety of conditions.

TGF-β1 has long been known to be a potent promoter of cartilage formation (see for example review by Kato [2]). In fact TGF-β1 has been used in implants to effect repair of cartilage in a number of studies, such as that of O'Driscoll [3], where it has been shown to induce chondrogenesis in tissues of mesenchymal origin (such as periosteum and muscle). There are also other reports of the use of TGF-β1 in osteochondral implants [4, 5]. Recently Brian Johnstone reported that TGF-β1, when added to primary cells in pellet culture, overcame the problem of lack of consistency and led to more reproducible results. This was an important development and has strengthened the original finding.

TGF-β3 has been found to have a pronounced effect on uterine leiomyoma cells [6]. Leiomyomas are benign smooth muscle tumors characterized by the formation of large amounts of extracellular matrix by hypertrophic cells with a low mitotic index. There is at least one reported case of leiomyoma showing a cartilage phenotype. Based on these observations we investigated the effect of TGF-β3 on chondrogenic differentiation of mesenchymal stem cells.

Human bone marrow-derived mesenchymal stem cells were cultured under standard conditions in the presence of 10% FBS until confluent. The cells were trypsinized, washed twice with chondrogenic medium, and resuspended at a density of 200,000 cells/ml in 15 ml polypropylene tubes in chondrogenic medium containing either 10 ng/ml TGF-β1, TGF-β3 or neither of these. The cells were spun to form a layer, the medium was replaced every 3-4 days and cells were harvested at 7, 14 and 21 days. Frozen sections (8 µm) were stained with Toluidine blue. They were also stained for the following using standard immunocytochemistry protocols: collagen type 11, collagen type 1, and cartilage oligomeric matrix protein (COMP).

Cells from the same donor were cultured in the chondrogenic medium with or without TGF-β1 or TGF-β3. On day zero (i.e., before spinning the cells to form a layer) type I collagen expression was observed in close proximity to the cells, but staining for type II collagenand COMP were absent. After 8 days in pellet culture in the absence of added growth factors COMP expression was abundant in the extracellular matrix, but type II collagen was not evident. In the presence of TGF-β1 there was still no expression of type II collagen. With TGF-β3 present a small area of the pellet had become acellular and some cells expressed type II collagen. At 14 days there was evidence of some chondrogenic differentiation in the presence of TGF-β1, as seen by the localized expression of type II collagen. When grown in the presence of TGF-β3 the pellets showed a dramatically different appearance, however. In this case the pellets were larger in size due to the presence of an abundant extracellular matrix. A large proportion of the cells were hypertrophic and there was expression of type II collagen throughout, except for a central area where the cells were still undifferentiated. Around the perimeter of the pellets the cells adopted an oblong configuration with intense type II staining, resembling perichondrium.

After 21 days in culture in the presence of TGF-β1 there was staining throughout the pellet except for the perimeter, which was negative. With TGF-β3 there was very strong staining, particularly in the interterritorial matrix. Staining with COMP was similar, with reduced pericellular staining and increased interterritorial staining.

This shows that TGF-β3 has a dramatic effect on chondrogenic differentiation of human MSCs *in vitro* and stimulates the development of abundant cartilage-like extracellular matrix. After 21 days in culture the tissue has a morphology that resembles mature articular cartilage. The specific expression of COMP in the interterritorial matrix is especially reminiscent of mature cartilage. In addition the abundant expression of type II collagen suggests that these cells have differentiated in a chondrogenic lineage. The effect is more pronounced in the presence of TGF-β3 compared to TGF-β1.

### Example 4

### Pellet Culture Experiment

### hMSC adherent culture

Human MSCs were isolated and expanded as previously described (Bruder et al., 1997) Growth kinetics, self-renewal and the osteogenic potential of purified human mesenchymal stem cells during extensive subcultivation and following cryopreservation. Journal of Cellular Biochemistry 64: 278-294, 1997. and grown as adherent cells. The growth medium was Dulbecco's Modified Eagle's Medium (DMEM) containing 1 g/l glucose, and supplemented with 10% fetal bovine serum from selected lots (Lennon et al., 1996) S.E. Haynesworth, S.P. Bruder, N. Jaiswal, and A.I. Caplan. Human and animal mesenchymal progenitor cells from bone marrow: identificationof serum for optimal selection and proliferation. In Vitro Cell. Dev. Biol.-Animal 32: 602-611, 1996. and 100 U/ml penicillin, 100 mg/ml streptomycin, and 250 ng/ml amphotericin B). Cells were grown in a 5% CO₂ atmosphere at 37° C. The cells were used at passage 1, corresponding to approximately 13 population doublings (Bruder et al., 1997). Growth kinetics, self-renewal and the osteogenic potential of purified human mesenchymal stem cells during extensive subcultivation and following cryopreservation. Journal of Cellular Biochemistry 64: 278-294, 1997.

### hMSC pellet culture

Chondrogenesis was promoted by switching hMSCs to a dense, 3-dimensional culture format, so that 250,000 cells formed a spheroidal pellet of about 1 mm initial diameter. To form these pellets, the adherent hMSCs were trypsinized, washed in serum-containing medium, then resuspended in serum-free chondrogenic medium, using standard tissue culture techniques. This medium was as described in Osiris Provisional Application #640100-129 [High-glucose DMEM supplemented with 10 ng/ml transforming growth factor beta-3 (TGF-β3, Oncogene Research Products, Cambridge, MA), 100 nM dexamethasone (Sigma, St. Louis, MO), 50 µg/ml ascorbic acid 2-phosphate (WAKO Pure Chemicals, Tokyo, Japan), 100 µg/ml sodium pyruvate, 40 µg/ml proline, and 6.25 µg/ml bovine insulin, 6.25 µg/ml transferrin, 6.25 µg/ml selenous acid, 5.33 µg/ml linoleic acid, 1.25 mg/ml bovine serum albumin (ITS-plus, Collaborative Biomedical Products, Cambridge, MA)].

Chondrogenic pellets of hMSCs were produced in the following manner. Aliquots of 250,000 cells suspended in 0.5 ml serum-free chondrogenic medium were distributed to 15 ml conical polypropylene centrifuge tubes (VWR). The cells were centrifuged for 5 minutes at 600 x g and left at the bottom of the tube. Tubes were placed in an incubator with caps loosened to permit gas exchange. The sedimented cells formed a spherical pellet at the bottom of the tube within 24 hours. Medium was replaced three times per week, and cells were cultured in this manner for up to four weeks.

### Induction of hypertrophy

Hypertrophic differentiation was induced after pellets had been cultured for 7 or 14 days under standard chondrogenic conditions. At that time point, the chondrogenic medium was replaced by a maturation medium containing 50 ng/ml thyroxine (Sigma). To further induce hypertrophy, the concentration of dexamethasone was lowered to 10⁻⁹ M (Quarto et al., 1992), and 20 mM β-glycerol phosphate (Sigma) was added (Dieudonne et al., 1994). C.M. Semeins, S.W. Goei, S. Vukicevic, J.K. Nulend, T.K. Sampath, M. Helder, and E.H. Burger. Opposite effects of osteogenic protein and transforming growth factor -b on chondrogenesis in cultured long bone rudiments. J. Bone Min. Res. 9: 771-780, 1994.

Other preliminary experiments indicate that T3 and thyroxine are both effective in inducing hypertrophy. Agents such as retinoic acid and its derivatives may also be effective in this fashion.

### Analysis of pellets

Cell pellets were harvested by fixation for I hr in 4% paraformaldehyde in PBS. Samples were then transferred into 70 % ethanol, dehydrated in ethanol and xylene series, and paraffin-embedded. 5 µm sections were cut through the center of each pellet.

Monospecific antibodies were used to detect extracellular matrix proteins characteristic of chondrogenic differentiation. Sections were digested for 30 min with 50 mU/ml chondroitinase ABC (Seikagaku America, Ijamsville, MD) in 100 mM Tris-acetate pH 7.6, 0.1 % bovine serum albumin. Type II collagen was identified with mouse monoclonal antibody C4F6, used at 2 mg/ml (Srinivas et al., 1993). Srinivas, G.R., H.J. Barrach, and C.O. Chichester. Quantitative immunoassays for type II collagen and its cyanogen bromide peptides. J. Immunol. Methods 159: 53-62, 1993 Types X and IX collagen were identified with mouse monoclonals X53 (Quartett, Berlin, FRG) (Girkontaite et al., 1996) S. Frischholz, P. Lammi, K. Wagner, B. Swoboda, T. Aigner, and K. Von der Mark. Immunolocalization of type X collagen in normal fetal and adult osteoarthritic cartilage with monoclonal antibodies. Matrix Biology 15: 231-238, 1996. and αCIX (C.O. Chichester et al., manuscript in preparation), respectively, after pepsin digestion. Prior to chondroitinase treatment, sections were incubated with 2 mg/ml pepsin (Sigma) in 0.5 M acetic acid for 1 hr. at 22 C.

For all immunostaining, reactivity was detected by serially incubating sections with biotinylated goat anti-mouse or anti-rabbit antibody, followed by streptavidin-horseradish peroxidase, according to the manufacturer's instructions (Kirkegaard & Perry Labs, Gaithersburg, MD). Signal was developed as the peroxidase reaction product of 3,3'-diaminobenzidine (DAB) and H₂O₂.

Anionic sulfated proteoglycans were detected by Safranin O staining and toluidine blue metachromasia. These stains characterize the deposition of a cartilaginous matrix (Sheehan and Hrapchak, 1980). Sheehan, D.C. and B.B. Hrapchak. Theory and Practice of Histochemistry, 2nd ed. Battelle Press, Columbus, OH. 481 pp. 1980.

### Example 5

### Sequential Treatment of hMSCs in Pellet Culture with Two Media Induces Hypertrophy

Human MSCs undergoing chondrogenic differentiation can mature into hypertrophic cells, either due to an internal program of gene expression or in response to environmental conditions. To induce hypertrophy, the conditions of *in vitro* culture were altered after two weeks of chondrogenic differentiation. Withdrawing TGF-β3 (Serra et al., 1997) Johnson, E.H. Filvaroff, J. LaBorde, D.M. Sheehan, R. Derynck, and H.L. Moses. Expressionof a truncated, kinase-defective TGF-β type II receptor in mouse skeletal tissue promotes terminal chondrocyte differentiation and osteoarthritis. J. Cell Biol. 139: 541-552, 1997. and adding 50 nM thyroxine (Ballock and Reddi, 1994) Thyroxine is the serum factor that regulates morphogenesis of columnar cartilage from isolated chondrocytes in chemically defined medium. J. Cell Biol. 126: 1311-18, 1994. to the media had a pronounced hypertrophic effect after two additional weeks of culture. Hypertrophy was more prominent when these conditions were combined with the addition of 20 mM β-glycerol phosphate and a reduction in the concentration of dexamethasone to 10⁻⁹ M. These pellets contained regions with hypertrophic cells surrounded by extensive pericellular accumulations of ECM (Fig. 2). Compared to pellets maintained in standard chondrogenic medium for 28 days, the treated pellets showed intense staining for type II and type IX collagens, as well as an irregular but pronounced increase in the deposition of type X collagen detected by antibody X53 (Fig. 2).

## Claims

1. A process for producing chondrocytes from mesenchymal stem cells by culturing mesenchymal stem cells in a chemically defined serum-free medium in vitro wherein the mesenchymal stem cells are associated in a three-dimensional format, and wherein said chemically defined serum-free medium comprises (1) a chemically defined minimum essential medium; (2) ascorbate; (3) an iron source; (4) insulin or an insulin-like growth factor; (5) at least one chondroinductive agent or factor; and (6) a simple sugar, said simple sugar being present in said medium in an amount of from 3g/l to 7 g/l.

2. The process of Claim 1 wherein said mesenchymal stem cells are isolated, culture expanded human mesenchymal stem cells.

3. The process of Claim 1 wherein the mesenchymal stem cells are condensed into close proximity.

4. The process of Claim 3 wherein the mesenchymal stem cells are present as packed cells or a centrifugal cell pellet.

5. The process of Claim 1 wherein said at least one chondroinductive agent or factor is selected from the group consisting of (i) a glucocorticoid; (ii) a member of the transforming growth factor-β super-family; (iii) a component of the collagenous extracellular matrix; and (iv) a vitamin A analog.

6. The process of Claim 1 wherein said at least one chondroinductive agent is a combination of dexamethasone and TGF-β1.

7. The process of Claim 1, and further comprising placing said cells in a rigid porous vessel.

8. The process of Claim 7 wherein said rigid porous vessel is a ceramic cube,

9. The process of Claim 1 wherein said simple sugar is glucose.

10. The process of Claim 9 wherein said glucose is present in said serum-free medium in an amount of 4.5 grams per liter.

11. The process of Claim 1 wherein at least one chondroinductive agent or factor comprises TGF-β3.

12. A process for inducing chondrogenesis in mesenchymal stem cells by culturing mesenchymal stem cells in a chemically defined serum-free medium in vitro wherein the mesenchymal stem cells are associated in a three-dimensional format, and wherein said chemically defined serum-free medium comprises (1) a chemically defined minimum essential medium; (2) ascorbate; (3) an iron source; (4) insulin or an insulin-like growth factor; (5) at least one chondroinductive agent or factor; and (6) a simple sugar, said simple sugar being present in said medium in an amount of from 3g/l to 7g/l.

13. The process of Claim 12 wherein said mesenchymal stem cells are isolated, culture expanded human mesenchymal stem cells.

14. The process of Claim 12 wherein the mesenchymal stem cells are condensed into close proximity.

15. The process of Claim 14 wherein the mesenchymal stem cells are present as packed cells or a centrifugal cell pellet.

16. The process of Claim 12 wherein said at least one chondroinductive agent is selected from the group consisting of (i) a glucocorticoid; (ii) a member of the transforming growth factor-β super-family; (iii) a component of the collagenous extracellular matrix; and (iv) a vitamin A analog.

17. The process of Claim 12 wherein said at least one chondroinductive agent is a combination of dexamethasone and TGF-β1.

18. The process of Claim 12, and further comprising placing said cells in a rigid porous vessel.

19. The process of Claim 18 wherein said rigid porous vessel is a ceramic cube.

20. The process of Claim 12 wherein said simple sugar is glucose.

21. The process of Claim 20 wherein said glucose is present in said serum-free medium in an amount of 4.5 grams per liter.

22. The process of Claim 12 wherein at least one chondroinductive agent or factor comprises TGF-β3.

## Patentansprüche

1. Verfahren zur Herstellung von Chondrozyten aus mesenchymalen Stammzellen durch in vitro-Züchtung von mesenchymalen Stammzellen in einem chemisch definierten, serumfreien Medium, wobei die mesenchymalen Stammzellen in einem dreidimensionalen Format assoziiert sind und wobei das chemisch definierte, serumfreie Medium folgendes umfasst: (1) ein chemisch definiertes Minimalmedium; (2) Ascorbat; (3) eine Eisenquelle; (4) Insulin oder einen insulinartigen Wachstumsfaktor; (5) mindestens ein chondroinduktives Mittel oder Faktor; und (6) einen einfachen Zucker, wobei der einfache Zucker im Medium in einer Menge von 3 g/Liter bis 7 g/Liter vorliegt.

2. Verfahren nach Anspruch 1, wobei es sich bei den mesenchymalen Stammzellen um isolierte, in Kultur expandierte, humane, mesenchymale Stammzellen handelt.

3. Verfahren nach Anspruch 1, wobei die mesenchymalen Stammzellen zu enger Nachbarschaft verdichtet sind.

4. Verfahren nach Anspruch 3, wobei die mesenchymalen Stammzellen als gepackte Zellen oder als zentrifugales Zellpellet vorliegen.

5. Verfahren nach Anspruch 1, wobei das mindestens eine chondroinduktive Mittel oder Faktor aus der Gruppe ausgewählt ist, die besteht aus (i) einem Glucocorticoid; (ii) einem Mitglied der transformierenden Wachstumsfaktor-β-Oberfamilie; (iii) einer Komponente der kollagenen, extrazellulären Matrix; und (iv) einem Vitamin A-Analogen.

6. Verfahren nach Anspruch 1, wobei es sich bei dem mindestens einen chondroinduktiven Mittel um eine Kombination von Dexamethason und TGF-β1 handelt.

7. Verfahren nach Anspruch 1, ferner umfassend das Platzieren der Zellen in ein starres, poröses Gefäß.

8. Verfahren nach Anspruch 7, wobei es sich bei dem starren, porösen Gefäß um einen Keramikwürfel handelt.

9. Verfahren nach Anspruch 1, wobei es sich bei dem einfachen Zucker um Glucose handelt.

10. Verfahren nach Anspruch 9, wobei die Glucose im serumfreien Medium in einer Menge von 4,5 g pro Liter vorliegt.

11. Verfahren nach Anspruch 1, wobei das mindestens eine chondroinduktive Mittel oder Faktor TGF-β3 umfasst.

12. Verfahren zur Induktion der Chondrogenese in mesenchymalen Stammzellen durch in vitro-Züchtung von mesenchymalen Stammzellen in einem chemisch definierten, serumfreien Medium, wobei die mesenchymalen Stammzellen in einem dreidimensionalen Format assoziiert sind und wobei das chemisch definierte, serumfreie Medium folgendes umfasst: (1) ein chemisch definiertes Minimalmedium; (2) Ascorbat; (3) eine Eisenquelle; (4) Insulin oder einen insulinartigen Wachstumsfaktor; (5) mindestens ein chondroinduktives Mittel oder Faktor; und (6) einen einfachen Zucker, wobei der einfache Zucker im Medium in einer Menge von 3 g/Liter bis 7 g/Liter vorliegt.

13. Verfahren nach Anspruch 12, wobei es sich bei den mesenchymalen Stammzellen um isolierte, in Kultur expandierte, humane, mesenchymale Stammzellen handelt.

14. Verfahren nach Anspruch 12, wobei die mesenchymalen Stammzellen zu enger Nachbarschaft verdichtet sind.

15. Verfahren nach Anspruch 14, wobei die mesenchymalen Stammzellen als gepackte Zellen oder als zentrifugales Zellpellet vorliegen.

16. Verfahren nach Anspruch 12, wobei das mindestens eine chondroinduktive Mittel aus der Gruppe ausgewählt ist, die besteht aus (i) einem Glucocorticoid; (ii) einem Mitglied der transformierenden Wachstumsfaktor-β-Oberfamilie; (iii) einer Komponente der kollagenen, extrazellulären Matrix; und (iv) einem Vitamin A-Analogen.

17. Verfahren nach Anspruch 12, wobei es sich bei dem mindestens einen chondroinduktiven Mittel um eine Kombination von Dexamethason und TGF-β1 handelt.

18. Verfahren nach Anspruch 12, ferner umfassend das Platzieren der Zellen in ein starres, poröses Gefäß.

19. Verfahren nach Anspruch 18, wobei es sich bei dem starren, porösen Gefäß um einen Keramikwürfel handelt.

20. Verfahren nach Anspruch 12, wobei es sich bei dem einfachen Zucker um Glucose handelt.

21. Verfahren nach Anspruch 20, wobei die Glucose im serumfreien Medium in einer Menge von 4,5 g pro Liter vorliegt.

22. Verfahren nach Anspruch 12, wobei das mindestens eine chondroinduktive Mittel oder Faktor TGF-β3 umfasst.

## Revendications

1. Procédé pour produire des chondrocytes à partir de cellules souches du mésenchyme par culture *in vitro* de cellules souches du mésenchyme dans un milieu chimiquement défini dépourvu de sérum, dans lequel les cellules souches du mésenchyme sont associées dans un format tridimensionnel, et dans lequel ledit milieu chimiquement défini dépourvu de sérum comprend (1) un milieu minimum essentiel chimiquement défini dépourvu de sérum ; (2) de l'ascorbate ; (3) une source de fer ; (4) de l'insuline ou un facteur de croissance analogue à l'insuline ; (5) au moins un agent ou facteur chondroinductif ; et (6) un sucre simple, ledit sucre simple étant présent dans ledit milieu selon une quantité de 3g/l à 7g/l.

2. Procédé suivant la revendication 1, dans lequel lesdites cellules souches du mésenchyme sont des cellules souches humaines du mésenchyme isolées, multipliées par culture.

3. Procédé suivant la revendication 1, dans lequel les cellules souches du mésenchyme sont condensées en une étroite proximité.

4. Procédé suivant la revendication 3, dans lequel les cellules souches du mésenchyme sont présentes sous forme de paquet cellulaire ou de culot cellulaire de centrifugation.

5. Procédé suivant la revendication 1, dans lequel ledit agent ou facteur chondroinductif est choisi parmi l'ensemble constitué par (i) un glucocorticoïde ; (ii) un membre de la super famille du facteur de croissance transformant β ; (iii) un composant de la matrice extracellulaire de collagène ; et (iv) un analogue de la vitamine A.

6. Procédé suivant la revendication 1, dans lequel ledit agent chondroinductif est une combinaison de dexaméthasone et de TGF-β1

7. Procédé suivant la revendication 1, comprenant en outre le placement desdites cellules dans un récipient rigide poreux.

8. Procédé suivant la revendication 7 dans lequel ledit récipient rigide poreux est un cube en céramique.

9. Procédé suivant la revendication 1 dans lequel ledit sucre simple est le glucose.

10. Procédé suivant la revendication 9 dans lequel ledit glucose est présent dans ledit milieu dépourvu de sérum selon une quantité de 4,5 grammes par litre.

11. Procédé suivant la revendication 1 dans lequel ledit agent ou facteur chondroinductif comprend TGF-β3

12. Procédé pour induire la chondrogenèse de cellules souches du mésenchyme par culture *in vitro* de cellules souches du mésenchyme dans un milieu chimiquement défini dépourvu de sérum, dans lequel les cellules souches du mésenchyme sont associées dans un format tridimensionnel, et dans lequel ledit milieu chimiquement défini dépourvu de sérum comprend (1) un milieu minimum essentiel chimiquement défini dépourvu de sérum ; (2) de l'ascorbate ; (3) une source de fer ; (4) de l'insuline ou un facteur de croissance analogue à l'insuline ; (5) au moins un agent ou facteur chondroinductif ; et (6) un sucre simple, ledit sucre simple étant présent dans ledit milieu selon une quantité de 3g/l à 7g/l.

13. Procédé suivant la revendication 12, dans lequel lesdites cellules souches du mésenchyme sont des cellules souches humaines du mésenchyme isolées, multipliées par culture.

14. Procédé suivant la revendication 12, dans lequel les cellules souches du mésenchyme sont condensées en une étroite proximité.

15. Procédé suivant la revendication 14, dans lequel les cellules souches du mésenchyme sont présentes sous forme de paquet cellulaire ou de culot cellulaire de centrifugation.

16. Procédé suivant la revendication 12, dans lequel ledit agent ou facteur chondroinductif est choisi parmi l'ensemble constitué par (i) un glucocorticoïde ; (ii) un membre de la super famille du facteur de croissance transformant β ; (iii) un composant de la matrice extracellulaire de collagène ; et (iv) un analogue de la vitamine A.

17. Procédé suivant la revendication 12, dans lequel ledit agent chondroinductif est une combinaison de dexaméthasone et de TGF-β1.

18. Procédé suivant la revendication 12, comprenant en outre le placement desdites cellules dans un récipient rigide poreux.

19. Procédé suivant la revendication 18 dans lequel ledit récipient rigide poreux est un cube en céramique.

20. Procédé suivant la revendication 12 dans lequel ledit sucre simple est le glucose.

21. Procédé suivant la revendication 20, dans lequel ledit glucose est présent dans ledit milieu dépourvu de sérum selon une quantité de 4,5 grammes par litre.

22. Procédé suivant la revendication 12 dans lequel ledit agent ou facteur chondroinductif comprend TGF-β3.
